# EUROPEAN PATENT SPECIFICATION

(11) **EP 3 236 951 B1**
(45) Date of publication and mention of the grant of the patent: **21.11.2018**
(21) Application number: 15820138.4
(22) Date of filing: 21.12.2015
(51) Int. Cl.: A61K 9/20, A61K 36/45, A61K 31/375, A61P 1/02

(54) **ORAL DISSOLVABLE PHARMACEUTICAL DOSAGE FORM FOR THE TREATMENT OF ORAL DISEASES**
IM MUND LÖSLICHE PHARMAZEUTISCHE DARREICHUNGSFORM ZUR BEHANDLUNG ORALER KRANKHEITEN
FORME POSOLOGIQUE PHARMACEUTIQUE À DISSOLUTION ORALE POUR LE TRAITEMENT DE MALADIES DE LA BOUCHE

(30) Priority: 22.12.2014 EP 14382556
(43) Date of publication of application: 01.11.2017
(73) Proprietor: Lacer, S.A., 08025 Barcelona (ES)
(72) Inventor: DE PABLO SEDANO, Marta, 08025 Barcelona (ES); DELGADO GAÑAN, Maria Isabel, 08025 Barcelona (ES)
(74) Representative: ZBM Patents - Zea, Barlocci & Markvardsen
(86) International application number: PCT/EP2015/080847
(87) International publication number: WO 2016/102502

(56) References cited:
- WO-A1-2009/141524
- US-A- 6 149 939
- US-A1- 2002 054 857
- US-A1- 2011 014 132
- US-A1- 2012 175 273
- US-B1- 7 771 756
- DATABASE WPI Section Ch, Week 200277 Thomson Scientific, London, GB; Class B05, AN 2002-709275 XP002740712, OTSUKI H; SEKIMOTO S: "Oral cavity dissolution tablet for preventing periodontal disease, comprises vitamins or calcium component as active ingredient, and sugar alcohol", -& JP 2002 121133 A ((SUNZ) SUNSTAR CHEM IND CO LTD) 23 April 2002 (2002-04-23)
- DATABASE WPI Section Ch, Week 200656 Thomson Scientific, London, GB; Class B04, AN 2006-544680 XP002740713, INUBUSHI J; SAITO T: "Inhibitor of dental bacteria co-aggregation useful in oral pharmaceuticals and foodstuffs for preventing and treating periodontal disease and caries, contains dry powder or solvent extract of linden, cinnamon, grape and rose", -& JP 2006 199661 A ((SUNZ) SUNSTAR CHEM IND CO LTD) 3 August 2006 (2006-08-03)
- DATABASE WPI Section Ch, Week 200544 Thomson Scientific, London, GB; Class B04, AN 2005-429917 XP002740801, JANG L J; LEE J J: "Composition useful as drink for preventing and removing hangover, contains crude drug extracts of Suaeda glauca, Inula britannica, Polygonum aviculare, Nettle and an additive", -& JP 2005 154411 A ((SALU-N) SALUS BIOTECH CO LTD-(SARU-N) SARUSU BIOTECH CORP) 16 June 2005 (2005-06-16)

## Description

The present invention relates to an orally disintegrating pharmaceutical dosage form comprising cranberry extract, and vitamin C. It also relates to the pharmaceutical dosage form for use in the prophylaxis and/or treatment of gingivitis or periodontitis.

### BACKGROUND ART

Gingivitis is a form of periodontal disease involving inflammation and infection that destroys the tissues that support the teeth, including the gums, the periodontal ligaments and the tooth sockets (alveolar bone).

Gingivitis is due to the long-term effects of plaque deposits on the teeth. Plaque is a sticky material made of bacteria, mucus and food debris that accumulates on the exposed areas of the tooth. If plaque is not avoided or eliminated, it turns into a hard deposit called tartar (or calculus) that becomes trapped at the base of the tooth. Plaque and tartar irritate and inflame the gums. Bacteria and the toxins they produce cause the gums to become infected, swollen, and sensitive. In the absence of treatment, gingivitis may progress to periodontitis, which is a destructive form of periodontal disease.

Currently there are multiple solutions either to prevent or to revert gingivitis, such as the use of toothpastes, mouthwashes, and gels containing broad spectrum antiseptics, anti-inflammatories, astringents, and so on. Nevertheless their effective application requires a correct and constant methodology by brushing the teeth three times a day for at least three minutes each time. This strict methodology requires a high degree of compliance, and therefore there is a high likelihood that the subject will not follow it with the required persistence.

It is known the ability of cranberry extract to prevent the adherence of bacteria to various structures of the mouth. WO2011162758 discloses a mouthwash formulation containing cranberry extract non-dialyzable material to inhibit bacterial co-aggregation in the oral cavity. US5683678 discloses a mouth rinse composition and a dentifrice composition comprising cranberry extract as antiplaque, anticalculus and anticaries agents. US2012175273 discloses a toothpaste tablet that dissolves quickly in the mouth prior to brushing. It contains (a) 0.1-10% wt of cranberry extract to reduce the levels of bacteria both in the oral cavity and on the toothbrush, (b) an abrasive based on dehydrated or hydrated silica to mechanically remove plaque and debris from the tooth surface, (c) an artificial sweetener to adjust the flavour of the tablet when chewed by a user, (d) a binder and (e) a surfactant.

It is also known that Vitamin C can be used in dental formulations in order to promote healing of the mouth from gum disease, and to reduce plaque buildup on the teeth. US20020054857 discloses toothpaste compositions comprising vitamin C and cranberry extract for reducing plaque adhesion to teeth and also for the treatment of gingivitis and periodontal disease.

Hence, although several formulations for the treatment of gingivitis have been proposed, there is still a need for a versatile, fast and effective product for the prevention and/or treatment of gingivitis.

### SUMMARY OF THE INVENTION

Inventors have found that a combination of a cranberry extract in a high percentage and vitamin C can be formulated as an orally disintegrating dosage form having a sufficient strength and being easy to handle without becoming sticky when stored or when it is merely put on the hand.

The pharmaceutical dosage form of the invention is adapted to supply the active ingredients to the oral cavity for the required period of time so that they can have a local action. Additionally, after being dissolved and swallowed, the active ingredients will be absorbed from the gastrointestinal tract for systemic distribution, thus enhancing their effectiveness.

Although formulations comprising cranberry extract and vitamin C that may be useful for the treatment of gingivitis have already been disclosed, their combination in a stable orally disintegrating dosage form with a double topical and systemic action has not been disclosed hitherto.

Thus, an aspect of the present invention is an orally disintegrating pharmaceutical dosage form comprising: cranberry extract in an amount from 3 to 18% by weight relative to the total weight of the composition, a therapeutically effective amount of vitamin C, a hydrophobic colloidal silicon dioxide, a diluent, and at least another pharmaceutically acceptable excipient or carrier.

The pharmaceutical composition of the invention is useful to prevent and/or to reduce formation of dental biofilm (plaque) on dental surface or on the gingival epithelial tissue, as well as to prevent and/or to treat the associated buccal-dental diseases, such as gingivitis or periodontitis.

Accordingly, another aspect of the invention is the orally disintegrating pharmaceutical dosage form as defined above for use in the prophylaxis and/or treatment of gingivitis or periodontitis.

### DETAILED DESCRIPTION OF THE INVENTION

The expression "therapeutically effective amount" as used herein, refers to the amount of a compound that, when administered, is sufficient to prevent development of, or alleviate to some extent, one or more of the symptoms of the disorder or condition being treated. The particular dose of compound administered according to this invention will of course be determined by the particular circumstances surrounding the case, including the particular condition being treated, the duration of the treatment, the nature of any concurrent treatment, the specific combination of active ingredient used, the particular excipients used and any other factor known to the expert.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ, particularly with the dental surface or the gingival epithelial tissue, of humans and animals without excessive toxicity, irritation, allergic response, immunogenicity or other problems or complications commensurate with a reasonable benefit/risk ratio.

The term "percentage by weight" or "wt%" refers to the percentage of each ingredient of the composition in relation to the total weight, unless otherwise stated.

The term "cranberry extract" is used herein in its conventional meaning to refer to concentrated preparations obtained using extraction procedures from cranberry (berries of *Vaccinium macrocarpon* and variants, such as *Vaccinium oxycoccos, Vaccinium microcarpum, and Vaccinium erythrocarpum*) using appropriate means. It can be obtained by any conventional technique, such as by concentration of the cranberry juice or by extraction of cranberry marc with water, an alcohol, such as ethanol, or a mixture thereof where appropriate. It is within the standard procedure of a skilled person to perform an appropriate extraction procedure in order to obtain an extract comprising the beneficial substances extracted from the cranberries. Document WO199526197 discloses a method for the preparation of a cranberry extract which includes the active fraction of cranberries responsible for the anti-bacterial adhesion activity. WO2009141524 discloses a process to obtain a cranberry extract from cranberry marc. The obtained extract is rich in proanthocyanidins, i.e. has an amount of proanthocyanidins from 1 to 50 wt%, particularly of type A proanthocyanidins, is soluble at various pH (and therefore in the saliva), and has antibacterial, antiviral, antioxidant and anti-adhesive properties. Particularly preferred are extracts from berries of *Vaccinium macrocarpon* and *Vaccinium oxycoccos.* Cranberry extract is also commercially available. As an instance, Nexira's *Exocyan* cranberry extract can be used.

Vitamin C, or L-ascorbic acid, can be in the dosage form as such or as any precursor or derivative form thereof. Thus, vitamin C refers to a number of vitamers that have vitamin C activity in animals, including humans, such as ascorbic acid itself and its salts, and some oxidized forms of the molecule like dehydroascorbic acid. A vitamer of a particular vitamin is any of a number of chemical compounds, generally having a similar molecular structure, each of which shows vitamin-activity in a vitamin-deficient biological system.

In a particular embodiment of the dosage form of the invention, optionally in combination with one or more features of the particular embodiments defined above or below, the cranberry extract is obtained by a process comprising the extraction from cranberry marc. Particularly, firstly, cranberry marc can be obtained by pressing cranberry berries, such as from the genus Vaccinium macrocarpon and/or Vaccinium oxycoccus, which allows removing a large proportion of the carbohydrates and organic acids present in the juice. Then, proanthocyanidins contained within the marc are extracted using an aqueous solution or a hydroalcoholic solution. In order to increase the extraction yield, the extracts can be passed through an adsorption column, such as of grafted silica resin, to better isolate proanthocyanidins. Absorption resin columns as disclosed in WO2009141524 can be used.

In another particular embodiment of the dosage form of the invention, optionally in combination with one or more features of the particular embodiments defined above or below, the cranberry is rich in proanthocyanidins, i.e. has an amount of proanthocyanidins from 1 to 50 wt%, particularly of proanthocyanidins of type A. More particularly, the cranberry extract comprises an amount of proanthocyanidins selected from 10, 20, 30, and 40% by weight.

As abovementioned, the amount of cranberry extract in the dosage form of the invention is from 3 to 18% by weight, more particularly from 4.5 to 6% by weight, relative to the total weight of the composition.

In a particular embodiment, optionally in combination with one or more features of the particular embodiments defined above or below, Vitamin C is present in an amount from 0.8% to 11% by weight, preferably of 8% by weight, relative to the total weight of the composition.

In another particular embodiment, optionally in combination with one or more features of the particular embodiments defined above or below, the invention relates to the dosage form defined above, wherein the weight ratio between cranberry extract and vitamin C is from 20 to 135 parts of cranberry extract, and from 6 to 80 parts of vitamin C. Particularly, the weight ratio between the two components is 3:4.

As abovementioned, the dosage form of the invention comprises a hydrophobic colloidal silicon dioxide. Hydrophobic colloidal silicon dioxide, such as, for example, Aerosil® R 972, allows the dosage form by forming a covering film on cranberry extract particles to be stabilized, so their hygroscopicity is reduced. Particularly, hydrophobic colloidal silicon dioxide is a fumed silica which has been surface modified with dimethyldichlorosilane. Generally speaking, hydrophobic silica is a form of silicon dioxide that has hydrophobic groups chemically bonded to the surface. Examples of these hydrophobic groups are alkyl or polydimethylsiloxane chains.

Suitable hydrophobic colloidal silicon dioxides include Aerosil® R 972, Aerosil® R 974, Aerosil® R 104, Aerosil® R 106, Aerosil® R 202, Aerosil® R 208, Aerosil® R 805, Aerosil® R 812, and Aerosil® 812 S, Aerosil® R 816, Aerosil® R 7200, Aerosil® R 8200, Aerosil® R 9200, and Aerosil® R 711, all commercialized by Evonik. Aerosil® R 972 and Aerosil® R 974 are particularly suitable. Other commercially available products are, for example, Cab-O-Sil TS 610 and Cab-O-Sil TS 620 (both Cabot).

Aerosil® R 972 is a fumed silicon dioxide treated with dimethyldichlorosilane (DDS) based on a hydrophilic fumed silica with a specific surface area of 130 m²/g. Aerosil® R 972 has a specific surface area (BET) of 90-130 m²/g, a pH value in 4% dispersion of 3.6-5.5, a loss on drying after 2 hours at 105 °C equal to or lower than 0.5 wt%, a carbon content of a 0.6-1.2 wt%, a tamped density (ISO 787-11, modified) of approximately 50 g/l, and a SiO₂ content based on ignited material equal to or lower than 99.8%. Aerosil® R 974 is a hydrophobic fumed silica treated with DDS based on a hydrophilic fumed silica with a specific surface area of 200 m²/g. Aerosil® R 974 has a specific surface area (BET) of 150-190 m²/g, a pH value in 4% dispersion of 3.7-4.7, a loss on drying after 2 hours at 105 °C equal to or lower than 0.5 wt%, a carbon content of a 0.9-1.5 wt%, a tamped density (ISO 787-11, modified) of approximately 50 g/l, and a SiO₂ content based on ignited material equal to or lower than 99.8%.

Hydrophobicity can be determined by various known methods such as the methanol wettability of Corning Glass (see "Methanol wettability acc. to Corning glass of hydrophobic AEROSIL® and AEROXIDE® products, PA 0420" in Analytical Test Method, Aerosil® of Evonik Industries published on October 2009), or the method for determination of the water-wettable contents as disclosed in WO2004039349, which is outlined below:

About 0.2 g of substance, weighed accurately to 0.001 g, are shaken intensively with 50 ml of water in a 250 ml pear-shaped separating funnel for 1 min. The funnel is then left to stand for one hour. During this, the predominant portion of the solid floats up. Without shaking up the suspension again, 45 ml of the liquid, which may be slightly cloudy, are drained off dropwise and transferred to a dish which has been dried at 140 °C and cooled in a desiccator. The liquid is evaporated off completely at 110-150 °C, during which it should be ensured that no substance sprays out. After cooling in a desiccator, the dish is weighed again. The weight difference with respect to the empty dish should be not more than 0.006 g. This corresponds to 3.0 wt. % of the substance weighed out. Hydrophobic colloidal silicon dioxide in which the water-wettable contents constitute a maximum of 3.0 wt% is particularly suitable for the dosage form of the invention.

In a particular embodiment, optionally in combination with one or more features of the particular embodiments defined above or below, the amount of hydrophobic colloidal silicon dioxide in the composition is from 0.2 to 5 wt%, particularly from 0.5 to 1% by weight, with respect to the total weight of the composition. In another particular embodiment, optionally in combination with one or more features of the particular embodiments defined above or below, the hydrophobic colloidal silicon dioxide is particularly present in the dosage form of the invention in an amount from 0.01 to 30 wt%, preferably in an amount from 0.1 to 20% by weight, more preferably in an amount from 3 to 10% by weight, relative to the weight of cranberry extract. The percentages apply to any one of the examples of suitable hydrophobic colloidal silicon dioxides abovementioned, namely, any one of the hydrophobic colloidal silicon dioxides can be present on their own in the amounts disclosed for the generic hydrophobic colloidal silicon dioxide.

As abovementioned, the dosage form of the invention comprises a diluent. In another particular embodiment, optionally in combination with one or more features of the particular embodiments defined above or below, the diluent is water-soluble. Examples of suitable water-soluble diluents include, without being limited to, alginates, calcium phosphate, calcium carbonate, cellulose powdered, cellulose microcrystalline, dextrates, dextrins, dextrose, fructose, isomalt, lactitol, lactose, mannitol, maltodextrin, maltose, polydextrose, polymethacrylates, sorbitol, and starch, and mixtures thereof.

Generally, the amount of diluent useful in accordance with the present invention can range from 30 to 90% by weight, preferably from 50 to 90% by weight, and more preferably from 60 to 80% by weight, relative to the total weight of the composition. The percentages apply to any one of the examples of diluents abovementioned, namely, any one of the diluents can be present on their own in the amounts disclosed for the generic diluent.

In a particular embodiment, optionally in combination with one or more features of the particular embodiments defined above or below, the diluent is mannitol, which helps to the modulation of the correct behaviour of cranberry extract in humid conditions.

In a particular embodiment, optionally in combination with one or more features of the particular embodiments defined above or below, the invention relates to an orally disintegrating pharmaceutical dosage form consisting of:
(i) cranberry extract in an amount from 3 to 18% by weight relative to the total weight of the composition,
(ii) a therapeutically effective amount of vitamin C,
(iiii) a hydrophobic colloidal silicon dioxide,
(iv) a diluent,
(v) at least another pharmaceutically acceptable excipient or carrier, and
(vi) optionally one or more components for buccal use having an effect other than preventing and/or reducing formation of dental biofilm on dental surface or on the gingival epithelial tissue, or preventing and/or treating gingivitis or periodontitis.

Examples of components for buccal use having an effect other than preventing and/or reducing formation of dental biofilm on dental surface or on the gingival epithelial tissue, or preventing and/or treating gingivitis or periodontitis, are, without being limited to, teeth whitening agents and teeth desensitizing agents.

Preferably, the total amount of cranberry extract comprising a 40% of proantocyanidins to be ingested per day is from 45 mg to 270 mg per day. The Recommended Dietary Allowance (RDA) of vitamin C is of 80 mg/day. Accordingly, the dosage form of the invention can contain from a 15% of the RDA of vitamin C, i.e. 12 mg, to a 200% of the RDA, i.e. 160 mg.

The RDA is defined as the level of intake of an essential nutrient that, on the basis of scientific knowledge, is judged by the Food and Nutrition Board or the Institute of Medicine of the National Academy of Sciences to be adequate to meet the known nutrient needs of practically all healthy persons.

The dosage form of the invention can be intended for one or more daily administrations. Preferably, a once daily dosing would avoid concerns about patient compliance.

Accordingly, in a preferred embodiment, optionally in combination with one or more features of the particular embodiments defined above or below, the dosage form of the invention is a once daily dosage form comprising from 45 to 270 mg of extract of cranberry (that is equivalent to an amount from 15 mg to 110 mg of proanthocyanidins, when the content of proanthocyanidins is of a 40 wt% of the weight of the extract), and from 12 to 200 mg of vitamin C.

In a more preferred embodiment, optionally in combination with one or more features of the particular embodiments defined above or below, the dosage form of the invention is a once daily dosage form comprising or consisting of 90 mg of cranberry extract (corresponding to an amount of 36 mg of proanthocyanidins when the content of proanthocyanidins is of a 40 wt% of the weight of the extract), and 120 mg of vitamin C, a hydrophobic colloidal silicon dioxide, a diluent, and other pharmaceutically acceptable excipients or carriers.

In a particular embodiment, optionally in combination with one or more features of the particular embodiments defined above or below, the dosage form of the invention further comprises xylitol. Xylitol, apart from having an antiseptic effect and providing some organoleptic properties to the composition, increases the adhesion of the particles, so increasing the breaking strength of the dosage form and diminishing their friability. Additionally, the combined effect of the hydrophobic colloidal silicon dioxide and xylitol allows obtaining a mixture suitable to be compressed, avoiding the wetting and drying steps in wet granulation, while the desired tablet hardness is achieved. Accordingly, particularly the orally disintegrating pharmaceutical dosage form is a tablet. In a particular embodiment, optionally in combination with one or more features of the particular embodiments defined above or below, the invention relates to an orally disintegrating pharmaceutical dosage form consisting of:
(i) cranberry extract in an amount from 3 to 18% by weight relative to the total weight of the composition,
(ii) a therapeutically effective amount of vitamin C,
(iiii) a hydrophobic colloidal silicon dioxide,
(iv) a diluent,
(v) xylitol,
(vi) at least another pharmaceutically acceptable excipient or carrier, and
(vii) optionally one or more components for buccal use having an effect other than preventing and/or reducing formation of dental biofilm on dental surface or on the gingival epithelial tissue, or preventing and/or treating gingivitis or periodontitis.

Generally, the amount of xylitol useful in accordance with the present invention can range from 5 to 40% by weight, preferably between 5 and 20% by weight, relative to the total weight of the composition.

Accordingly, in a particular embodiment, optionally in combination with one or more features of the particular embodiments defined above or below, the present invention relates to the dosage form defined above, wherein the weight ratio between cranberry extract, vitamin C and xylitol is from 20 to 135 parts of cranberry extract, from 6 to 80 parts of vitamin C, and from 37,5 to 300 parts of xylitol. Particularly, the weight ratio among the three components is 6:8:5-40, particularly 6:8:5-20, more particularly 6:8:5, 6:8:20, and 6:8:40.

In a more preferred embodiment, optionally in combination with one or more features of the particular embodiments defined above or below, the dosage form of the invention is a once daily dosage form comprising or consisting of 90 mg of cranberry extract, and 120 mg of vitamin C, and from 0 to 600 mg of xylitol, particularly from 75 to 300 mg of xylitol, and more particularly 75 mg of xylitol, a hydrophobic colloidal silicon dioxide, a diluent, and other pharmaceutically acceptable excipients or carriers.

In another particular embodiment, optionally in combination with one or more features of the particular embodiments defined above or below, the dosage form of the invention further comprises a taste-masking agent in an amount sufficient to mask the bitter taste of cranberry extract.

The taste-masking agent is present in an amount sufficient to mask the unpleasant bitter taste of the cranberry extract when it is used at the high amounts of the dosage form of the invention. Examples of suitable taste-masking agents include, without being limited to, vanillin, ethylvanillin, and ethylmaltol.

In a particular embodiment, optionally in combination with one or more features of the particular embodiments defined above or below, the taste masking agent is present in an amount from 0.1% to 3% by weight, preferably from 0.1% to 0,15% by weight, relative to the total weight of the composition. The percentages apply to any one of the examples of suitable taste-masking agents abovementioned, namely, any one of the taste-masking agents can be present in the amounts disclosed for the generic taste-masking agent.

In a particular embodiment, optionally in combination with one or more features of the particular embodiments defined above or below, the invention relates to an orally disintegrating pharmaceutical dosage form consisting of:
(i) cranberry extract in an amount from 3 to 18% by weight relative to the total weight of the composition,
(ii) a therapeutically effective amount of vitamin C,
(iiii) a hydrophobic colloidal silicon dioxide,
(iv) a diluent,
(v) xylitol,
(vi) a taste masking agent, and
(vii) at least another pharmaceutically acceptable excipient or carrier, and
(viii) optionally one or more components for buccal use having an effect other than preventing and/or reducing formation of dental biofilm on dental surface or on the gingival epithelial tissue, or preventing and/or treating gingivitis or periodontitis.

In another particular embodiment, optionally in combination with one or more features of the particular embodiments defined above or below, the taste masking agent is vanillin. The use of the taste-masking agent allows avoiding the problems derived from the bitterness of cranberry extract at high concentrations.

In a particular embodiment, optionally in combination with one or more features of the particular embodiments defined above or below, the present invention relates to the dosage form defined above further comprising a flavour, which in combination with the taste-masking agent produces a more pleasant taste dosage form.

Examples of suitable flavours include, without being limited to, cola flavour, and fruit essences, such as strawberry, raspberry, cherry, and blackberry, and mixtures thereof. The amount of flavouring may depend on a number of factors, including the organoleptic effect desired. Flavours may be present in an amount ranging from 0.05 to 10 percent by weight based upon the weight of the total composition. Particularly preferred flavours are 2 wt%. The percentages apply to any one of the examples of suitable flavours abovementioned, namely, any one of the flavours can be present in the amounts disclosed for the generic flavour.

In a preferred embodiment, optionally in combination with one or more features of the particular embodiments defined above or below, the present invention relates to the dosage form defined above that, in addition to the taste-masking agent and the flavour as defined above, further comprises a sweetener to produce even a more pleasant taste composition.

In a particular embodiment, optionally in combination with one or more features of the particular embodiments defined above or below, the invention relates to an orally disintegrating pharmaceutical dosage form consisting of:
(i) cranberry extract in an amount from 3 to 18% by weight relative to the total weight of the composition,
(ii) a therapeutically effective amount of vitamin C,
(iiii) a hydrophobic colloidal silicon dioxide,
(iv) a diluent,
(v) xylitol,
(vi) a taste masking agent,
(vii) at least another pharmaceutically acceptable excipient or carrier, wherein at least one of the pharmaceutically acceptable excipients or carriers is a flavour, a sweetener, or a combination thereof, and
(viii) optionally one or more components for buccal use having an effect other than preventing and/or reducing formation of dental biofilm on dental surface or on the gingival epithelial tissue, or preventing and/or treating gingivitis or periodontitis.

Examples of sweeteners include, without being limited to, acesulfame potassium, aspartame, cyclamic acid and the sodium and calcium salts thereof, saccharine and the sodium and calcium salts thereof, sucralose, thaumatin, neohesperidin dihydrochalcone, neotame, and stevia.

In another particular embodiment, optionally in combination with one or more features of the particular embodiments defined above or below, the invention relates to the dosage form defined above, wherein the weight ratio among taste-masking agent, flavour, and sweetener is from 0 to 5 parts of taste-masking agent, from 0 to 10 parts of flavour, and from 0 to 40 parts of sweetener. More preferably the taste-masking agent is vanillin and the weight ratio among taste-masking agent, flavour, and sweetener is 1:5-8:4-15, particularly 1:5-8:6-10.

In a more preferred embodiment, optionally in combination with one or more features of the particular embodiments defined above or below, the dosage form of the invention is a once daily dosage form comprising or consisting of 90 mg of cranberry extract, and 120 mg of vitamin C, and from 0 to 600 mg of xylitol, particularly from 50 to 300 mg of xylitol, and more particularly 75 mg of xylitol, a taste masking agent, a flavour, a sweetener, a hydrophobic colloidal silicon dioxide, a diluent, and other pharmaceutically acceptable excipients or carriers.

As abovementioned, the dosage form of the invention also comprises other suitable pharmaceutically acceptable excipients or carriers, which include, without being limited to, flow improving agents, anti-adherent agents, and lubricants.

Examples of flow improving and anti-adherent agents include, without being limited to, hydrophilic colloidal silicon dioxide, such as, for example, Aerosil® 200, which is used to provide the desirable flow characteristics.

Suitable hydrophilic colloidal silicon dioxide include Aerosil® 90, Aerosil® 130, Aerosil® 150, Aerosil® 200, Aerosil® 255, Aerosil® 300, Aerosil® 380, Aerosil® OX 50, and Aerosil® TT 600, Aerosil® 200 F, Aerosil® 380 F, Aerosil® 200 Pharma, Aerosil® 300 Pharma, and Aeroperl® 300/30, and Aeroperl® 300 Pharma, all commercialized by Evonik. Aerosil® 200 Pharma and Aerosil® 200 are particularly suitable.

Particularly, the hydrophilic colloidal silicon dioxide can be present in the dosage form of the invention in an amount from 0.5 to 1% by weight, preferably in an amount of 0.8% by weight, relative to the total weight of the composition. The percentages apply to any one of the examples of hydrophilic colloidal silicon dioxides abovementioned, namely, any one of the hydrophilic colloidal silicon dioxides can be present on their own in the amounts disclosed for the generic hydrophilic colloidal silicon dioxide.

Examples of lubricants include, without being limited to, calcium stearate, glycerin monostearate, glycerin palmitostearate, hydrogenated castor oil, hydrogenated vegetable oil, magnesium lauryl sulfate, magnesium stearate, medium chain triglycerides, mineral oil, myristic acid, palmitic acid, polyethylene glycol, potassium benzoate, sodium benzoate, stearic acid, and talc.

The amount of lubricant used can generally range from 0.5 to 4 by weight, and more preferably from 1.5 to 4% by weight, relative to the total weight of the composition. The percentages apply to any one of the examples of lubricants abovementioned, namely, any one of the lubricants can be present on their own in the amounts disclosed for the generic lubricant.

The disintegrating rate of the pharmaceutical composition formulated as an orally disintegrating dosage form is a considerably important parameter for the dual topical and systemic effect of the active ingredients and the effectiveness of the treatment. The inventors have observed that the specific combination of the abovementioned amounts of diluent agent, colloidal silicon dioxide, lubricants, and, optionally, xylitol has an influence on the disintegrating rate of the obtained dosage form.

Accordingly, in a particular embodiment, optionally in combination with one or more features of the particular embodiments defined above or below, the invention relates to the dosage form defined above, which is capable of disintegrating in the mouth in a period of time from 2 to 15 minutes. Particularly, in the dosage form of the invention as defined above, the amount of mannitol can be from 30 to 90% by weight, preferably between 50 and 90% by weight, and more preferably from 60 to 80% by weight; the amount of hydrophilic colloidal silicon dioxide can be from 0.5 to 1% by weight, preferably of 0.8% by weight; the amount of lubricant can be from 0.5 to 4% by weight, and more preferably from 1.5 to 4% by weight; and the amount of xylitol can be from 5 to 40% by weight, preferably between 5 and 20% by weight, relative to the total weight of the composition; provided their total does not exceed the 84% of the weight of the composition. More particularly, the amount of mannitol can be from 30 to 90% by weight; the amount of hydrophilic colloidal silicon dioxide can be from 0.5 to 1% by weight, the amount of lubricant can be from 1 to 2.5% by weight, and the amount of xylitol can be from 5 to 40% by weight, relative to the total weight of the composition; provided their total does not exceed the 84% of the weight of the composition.

In the context of the invention, it is understood that a particularly preferred orally disintegrating pharmaceutical dosage form of the invention is a tablet. Generally, the compression force is adjusted in order to obtain a tablet with a hardness from 100 N to 200 N providing the desired disintegrating rate in the mouth, namely from 2 to 15 minutes.

The disintegration time in the mouth can be measured by timing how long it takes for the tablet to become completely dispersed in the mouth after being put on top of the tongue and melt without being chewed.

The orally disintegrating pharmaceutical dosage form of the invention can be prepared as disclosed below in the examples, all of them relating to orally disintegrating tablets. During the compression process, parameters such as the aspect, mass, hardness, height, and friability of the tablets are controlled. Direct compression is the preferred method of forming the dosage forms.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### EXAMPLE 1

- Phase I: Sieving the cranberry extract, and optionally the xylitol, together with the silicas, the diluent (mannitol), at least one of the flavours, and half the amount of magnesium stearate, through a 0.6 mm screen, and mixing the components for 20 minutes;
- Phase II: Separately, sieving the ascorbic acid, the sweeteners, together with the rest of flavours, and the lubricant (e.g. talc) through a 0.6 mm, and mixing the components for 5 min;
- Mixing the two obtained sievings, and adding the other half amount of magnesium stearate, previously sieved through a 0.6 mm sieving, for 5 minutes;
- Compressing the final mixture on a rotatory compression machine to form the orally disintegrating tablet by direct compression.

| **Components** | **mg/comp.** |
|---|---|
| **Phase I** | |
| Cranberry 3 | 90,0 |
| Mannitol DC 300 | 1094,0 |
| Xylitol Farma | 75,0 |
| Vainillin | 1,0 |
| Cocacola flavour | 15,0 |
| Aerosil 200 | 12,0 |
| Aerosil R972 | 3,0 |
| Mg estearate | 22,0 |

| **Phase II** | |
|---|---|
| Na saccharin | 20,0 |
| Aspartame | 10,0 |
| Ascorbic acid | 120,0 |
| Talc | 15,0 |
| Mg estearate | 22,0 |
| **TOTAL** | **1499,0** |

### EXAMPLE 2

The tablet was obtained following the process of Example 1.

| **Components** | **mg/comp.** |
|---|---|
| **Phase I** | |
| Cranberry 3 | 90,0 |
| Mannitol DC 300 | 1079,0 |
| Xylitol Farma | 75,0 |
| Vainillin | 2,0 |
| Aerosil 200 | 12,0 |
| Aerosil R972 | 3,0 |
| Mg estearate | 22,0 |

| **Phase II** | |
|---|---|
| Blackberry flavour | 30,0 |
| Na saccharin | 20,0 |
| Aspartame | 10,0 |
| Ascorbic acid | 120,0 |
| Talc | 15,0 |
| Mg estearate | 22,0 |
| **TOTAL** | **1500,0** |

### EXAMPLE 3

The tablet was obtained following the process of Example 1.

| **Components** | **mg/comp.** |
|---|---|
| **Phase I** | |
| Cranberry 3 | 90,0 |
| Mannitol DC 300 | 1079,0 |
| Xylitol Farma | 75,0 |
| Vainillin | 2,0 |
| Aerosil 200 | 12,0 |
| Aerosil R972 | 3,0 |
| Mg estearate | 22,0 |

| **Phase II** | |
|---|---|
| Blackberry flavour | 30,0 |
| Na saccharin | 20,0 |
| Neohersperidina | 10,0 |
| Ascorbic acid | 120,0 |
| Talc | 15,0 |
| Mg estearate | 22,0 |
| **TOTAL** | **1500,0** |

### EXAMPLE 4

The tablet was obtained following the process of Example 1.

| **Components** | **mg/comp.** |
|---|---|
| **Phase I** | |
| Cranberry 3 | 90,0 |
| Mannitol DC 300 | 1102,7 |
| Xylitol Farma | 75,0 |
| Vainillin | 2,0 |
| Aerosil 200 | 12,0 |
| Aerosil R972 | 3,0 |
| Mg estearate | 22,0 |

| **Phase II** | |
|---|---|
| Blackberry flavour | 30,0 |
| Na saccharin | 2,3 |
| Sucralose | 3,5 |
| Neohersperidina | 0,5 |
| Ascorbic acid | 120,0 |
| Talc | 15,0 |
| Mg estearate | 22,0 |
| **TOTAL** | **1500,0** |

### EXAMPLE 5

The tablet was obtained following the process of Example 1.

| **Components** | **mg/comp.** |
|---|---|
| **Phase I** | |
| Cranberry 3 | 90,0 |
| Mannitol DC 300 | 1106,9 |
| Xylitol Farma | 75,0 |
| Vainillin | 2,0 |
| Aerosil 200 | 12,0 |
| Aerosil R972 | 3,0 |
| Mg estearate | 22,0 |

| **Phase II** | |
|---|---|
| Blackberry flavour | 30,0 |
| Na saccharin | 0,75 |
| Sucralose | 1,2 |
| Neohersperidina | 0,15 |
| Ascorbic acid | 120,0 |
| Talc | 15,0 |
| Mg estearate | 22,0 |
| **TOTAL** | **1500,0** |

### EXAMPLE 6

The tablet was obtained following the process of Example 1.

| **Components** | **mg/comp.** |
|---|---|
| **Phase I** | |
| Cranberry 3 | 90,0 |
| Mannitol 2 | 570,0 |
| Xylitol 1 | 600,0 |
| Flavour 2 (coca-cola) | 15,0 |
| Aerosil 2 (200) | 7,5 |
| Aerosil 1 (R792) | 7,5 |
| Mg estearate | 22,0 |

| **Phase II** | |
|---|---|
| Na saccharin | 20,0 |
| Aspartame | 10,0 |
| Ascorbic acid | 120,0 |
| Talc | 15,0 |
| Mg estearate | 22,0 |
| **TOTAL** | **1500,0** |

### EXAMPLE 7

The tablet was obtained following the process of Example 1.

| | |
|---|---|
| **Components** | **mg/comp.** |

| **Phase I** | |
|---|---|
| Cranberry 3 | 90,0 |
| Mannitol 2 | 1170,0 |
| Flavour 1 (Vainille) | 1,0 |
| Flavour 2 (coca-cola) | 15,0 |
| Aerosil 2 (200) | 12,0 |
| Aerosil 1 (R792) | 3,0 |
| Mg estearate | 22,0 |

| **Phase II** | |
|---|---|
| Na saccharin | 20,0 |
| Aspartame | 10,0 |
| Ascorbic acid | 120,0 |
| Talc | 15,0 |
| Mg estearate | 22,0 |
| **TOTAL** | **1500,0** |

### EXAMPLE 8

The tablet was obtained following the process of Example 1.

| **Components** | **mg/comp.** |
|---|---|
| **Phase I** | |
| Cranberry 3 | 90,0 |
| Mannitol 2 | 810,0 |
| Xylitol 1 | 300,0 |
| Flavour 1 (Vainilla) | 1,0 |
| Flavour 2 (coca-cola) | 15,0 |
| Aerosil 2 (200) | 12,0 |
| Aerosil 1 (R792) | 3,0 |
| Polietilenglicol | 75,0 |
| Mg estearate | 22,0 |

| **Phase II** | |
|---|---|
| Aspartame | 15,0 |
| Ascorbic acid | 120,0 |
| Talc | 15,0 |
| Mg estearate | 22,0 |
| **TOTAL** | **1500,0** |

### EXAMPLE 9

The tablet was obtained following the process of Example 1.

| **Components** | **mg/comp.** |
|---|---|
| **Phase I** | |
| Cranberry 3 | 90,0 |
| Mannitol 2 | 1105,0 |
| Xylitol 1 | 75,0 |
| Flavour 1 (Vainilla) | 1,0 |
| Flavour 2 (coca-cola) | 15,0 |
| Aerosil 2 (200) | 12,0 |
| Aerosil 1 (R792) | 3,0 |
| Mg estearate | 22,0 |

| **Phase II** | |
|---|---|
| Aspartame | 20,0 |
| Ascorbic acid | 120,0 |
| Talc | 15,0 |
| Mg estearate | 22,0 |
| **TOTAL** | **1500,0** |

### EXAMPLE 10. In vivo assay

The tissue response to the orally disintegrating tablet of Example 3 and its clinical efficacy was evaluated after 21 days of daily use (one tablet per day) in 21 subjects (males and females) with experimentally induced gingivitis.

As a control, a placebo with the same composition as the orally disintegrating tablets mentioned above but without cranberry, xylitol and ascorbic acid was used.

Subjects were recommended to take the tablet at night after dinner and after an oral rinse with tap water. Subsequently, the tablet is left in the mouth until complete dissolution. Subjects were recommended not to drink or eat food before a minimum of 30 minutes after the dissolution of the tablet.

The following parameters were assessed:
- Loë & Silness gingival index (GI)
- Gingival bleeding index (GBI)
- Interleukins level assessment

A pilot prospective randomized double-blind, placebo-controlled was carried out. A basic dental cleaning was carried out in the selected population to homogenize sample to the maximum. Subsequently, subjects participating in the study were randomized to either the experimental group or the control (placebo) group. The appearance of the medication and dosage were maintained. Measurements of the above mentioned parameters were performed at 0, 15 and 21 days.

### Results

### Loë & Silness gingival index (GI)

0 Indicates less rate of gingival inflammation, the closer to 0 the better. The experimental group presented a GI (0.97 ± 0.54), which was statistically significantly lower (p = 0.0001) than the control group (1.48 ± 0.60).

### Gingival bleeding index (GBI)

0 Indicates less bleeding index, the closer to 0 the better. The experimental group presented a GBI (0.98 ± 0.67), which was statistically significantly lower (p = 0.0001) than the control group (1.61 ± 0.66).

### Interleukins level assessment (IL6-IL8; marker of inflammation)

A significant decrease in IL6 measurements in the experimental group compared to the control group 15 (p = 0.019) and 21 days (p = 0.011) in crevicular fluid was observed. Data obtained for IL8 were equivalent.

## Claims

1. An orally disintegrating pharmaceutical dosage form comprising
- cranberry extract in an amount from 3 to 18% by weight relative to the total weight of the composition,
- a therapeutically effective amount of vitamin C,
- a hydrophobic colloidal silicon dioxide,
- a diluent, and
- at least another pharmaceutically acceptable excipient or carrier.

2. The dosage form according to claim 1, which is a tablet.

3. The dosage form according to claim 2, wherein the tablet has a hardness from 100 N to 200 N, and is capable of disintegrating in the mouth in a period of time from 2 to 15 minutes.

4. The dosage form according to any one of claims 1 to 3, wherein the weight ratio between cranberry extract and vitamin C is from 20 to 135 parts of cranberry extract, and from 6 to 80 parts of vitamin C.

5. The dosage form according to any one of claims 1 to 4, wherein the hydrophobic colloidal silicon dioxide is in an amount from 0.01 to 30 wt% with respect to the amount of cranberry extract.

6. The dosage form according to any one of claims 1 to 5, wherein the diluent is mannitol.

7. The dosage form according to any one of claims 1 to 6, which is a once-daily dosage form comprising from 45 to 270 mg of extract of cranberry, and from 12 to 200 mg of vitamin C.

8. The dosage form according to claim 7, comprising 90 mg of cranberry extract, and 120 mg of vitamin C.

9. The dosage form according to any one of claims 1 to 8, further comprising xylitol.

10. The dosage form according to claim 9, wherein the amount of xylitol ranges from 5 to 40% by weight relative to the total weight of the composition.

11. The dosage form according to any one of claims 1 to 10, wherein the dosage form further comprises a taste-masking agent in an amount sufficient to mask the bitter taste of cranberry extract.

12. The dosage form according to claim 11, wherein the taste masking agent is present in an amount from 0.1% to 3% by weight relative to the total weight of the composition.

13. The dosage form according to any one of claims 11 to 12, wherein the taste-masking agent is vanillin.

14. The dosage form according to any one of claims 1 to 13, further comprising hydrophilic colloidal silicon dioxide in an amount from 0.5 to 1% by weight relative to the total weight of the composition.

15. The orally disintegrating pharmaceutical dosage form as defined in any one of claims 1 to 14, for use in the prophylaxis and/or treatment of gingivitis or periodontitis.

## Patentansprüche

1. Eine sich im Mund auflösende pharmazeutische Arzneiform umfassend
- Cranberryextrakt in einer Menge von 3 bis 18 Gew.-% bezogen auf das gesamte Gewicht der Zusammensetzung,
- eine therapeutisch wirksame Menge von Vitamin C,
- ein hydrophobes kolloidales Siliziumdioxid,
- ein Streckmittel, und
- mindestens einen weiteren pharmazeutisch akzeptablen Hilfsstoff oder Trägerstoff.

2. Die Arzneiform nach Anspruch 1, welche eine Tablette ist.

3. Die Arzneiform nach Anspruch 2, wobei die Tablette eine Härte von 100 N bis 200 N hat und sich im Mund innerhalb eines Zeitraums von 2 bis 15 Minuten auflösen kann.

4. Die Arzneiform nach einem der Ansprüche 1 bis 3, wobei das Gewichtsverhältnis von Cranberryextrakt und Vitamin C von 20 bis 135 Teilen von Cranberryextrakt und von 6 bis 80 Teilen von Vitamin C reicht.

5. Die Arzneiform nach einem der Ansprüche 1 bis 4, wobei das hydrophobe kolloidale Siliziumdioxid in einer Menge von 0,01 bis 30 Gew.-% bezogen auf die Menge von Cranberryextrakt vorhanden ist.

6. Die Arzneiform nach einem der Ansprüche 1 bis 5, wobei das Streckmittel Mannitol ist.

7. Die Arzneiform nach einem der Ansprüche 1 bis 6, welche eine einmal täglich einzunehmende Arzneiform umfassend von 45 bis 270 mg Cranberryextrakt und von 12 bis 200 mg Vitamin C ist.

8. Die Arzneiform nach Anspruch 7 umfassend 90 mg Cranberryextrakt und 120 mg Vitamin C.

9. Die Arzneiform nach einem der Ansprüche 1 bis 8, weiterhin umfassend Xylitol.

10. Die Arzneiform nach Anspruch 9, wobei die Menge von Xylitol von 5 bis 40 Gew.-% bezogen auf das gesamte Gewicht der Zusammensetzung reicht.

11. Die Arzneiform nach einem der Ansprüche 1 bis 10, wobei die Arzneiform weiterhin einen Geschmacksmaskierer in einer zur Maskierung des bitteren Geschmacks von Cranberryextrakt ausreichenden Menge umfasst.

12. Die Arzneiform nach Anspruch 11, wobei der Geschmacksmaskierer in einer Menge von 0,1 Gew.-% bis 3 Gew.-% bezogen auf das gesamte Gewicht der Zusammensetzung vorhanden ist.

13. Die Arzneiform nach einem der Ansprüche 11 bis 12, wobei der Geschmacksmaskierer Vanillin ist.

14. Die Arzneiform nach einem der Ansprüche 1 bis 13, weiterhin umfassend hydrophiles kolloidales Siliziumdioxid in einer Menge von 0,5 bis 1 Gew.-% bezogen auf das gesamte Gewicht der Zusammensetzung.

15. Die sich im Mund auflösende pharmazeutische Arzneiform wie in einem der Ansprüche 1 bis 14 definiert zur Verwendung bei der Prophylaxe und/oder der Behandlung der Gingivitis oder der Parodontitis.

## Revendications

1. Une forme galénique pharmaceutique à désintégration orale comprenant
- de l'extrait de canneberge dans une quantité allant de 3 à 18 % en poids par rapport au poids total de la composition,
- une quantité thérapeutiquement efficace de vitamine C,
- un dioxyde de silicium colloïdal hydrophobe,
- un diluant, et
- au moins un autre excipient ou véhicule pharmaceutiquement acceptable.

2. La forme galénique selon la revendication 1, qui est un comprimé.

3. La forme galénique selon la revendication 2, dans laquelle le comprimé a une dureté allant de 100 N à 200 N et peut se désintégrer dans la bouche dans une période de temps allant de 2 à 15 minutes.

4. La forme galénique selon l'une quelconque des revendications 1 à 3, dans laquelle le rapport en poids entre l'extrait de canneberge et la vitamine C va de 20 à 135 parties d'extrait de canneberge et de 6 à 80 parties de vitamine C.

5. La forme galénique selon l'une quelconque des revendications 1 à 4, dans laquelle le dioxyde de silicium colloïdal hydrophobe est dans une quantité allant de 0,01 à 30 % en poids par rapport à la quantité d'extrait de canneberge.

6. La forme galénique selon l'une quelconque des revendications 1 à 5, dans laquelle le diluant est le mannitol.

7. La forme galénique selon l'une quelconque des revendications 1 à 6, qui est une forme galénique à administrer une fois par jour comprenant de 45 à 270 mg d'extrait de canneberge et de 12 à 200 mg de vitamine C.

8. Le forme galénique selon la revendication 7, comprenant 90 mg d'extrait de canneberge et 120 mg de vitamine C.

9. La forme galénique selon l'une quelconque des revendications 1 à 8, comprenant en outre du xylitol.

10. La forme galénique selon la revendication 9, dans laquelle la quantité de xylitol va de 5 à 40 % en poids par rapport au poids total de la composition.

11. La forme galénique selon l'une quelconque des revendications 1 à 10, dans laquelle la forme galénique comprend en outre un agent de masquage du goût dans une quantité suffisante pour masquer le goût amer de l'extrait de canneberge.

12. La forme galénique selon la revendication 11, dans laquelle l'agent de masquage du goût est présent dans une quantité allant de 0,1 % à 3 % en poids par rapport au poids total de la composition.

13. La forme galénique selon l'une quelconque des revendications 11 à 12, dans laquelle l'agent de masquage du goût est la vanilline.

14. La forme galénique selon l'une quelconque des revendications 1 à 13, comprenant en outre du dioxyde de silicium colloïdal hydrophile dans une quantité allant de 0,5 à 1 % en poids par rapport au poids total de la composition.

15. La forme galénique pharmaceutique à désintégration orale telle que définie dans l'une quelconque des revendications 1 à 14, pour l'utilisation dans la prophylaxie et/ou le traitement de la gingivite ou la parodontite.
